# EUROPEAN PATENT SPECIFICATION

(11) **EP 3 052 941 B1**
(45) Date of publication and mention of the grant of the patent: **27.03.2019**
(21) Application number: 14777609.0
(22) Date of filing: 30.09.2014
(51) Int. Cl.: G01N 33/68

(54) **A METHOD FOR PREDICTING THE RISK OF GETTING A MAJOR ADVERSE CARDIAC EVENT**
VERFAHREN ZUR VORHERSAGE DES RISIKOS EINES UNERWÜNSCHTEN HERZEREIGNISSES
PROCÉDÉ PERMETTANT DE PRÉVOIR LES RISQUES D'OBTENIR UN ÉVÉNEMENT CARDIAQUE INDÉSIRABLE MAJEUR

(30) Priority: 01.10.2013 EP 13186938
(43) Date of publication of application: 10.08.2016
(73) Proprietor: SphingoTec GmbH, 16761 Hennigsdorf (DE)
(72) Inventor: BERGMANN, Andreas, 13465 Berlin (DE); NG, Leong, Leicester Leicestershire LE1 7RH (GB)
(74) Representative: Kilger, Ute
(86) International application number: PCT/EP2014/070962
(87) International publication number: WO 2015/049243

(56) References cited:
- WO-A1-2010/128071
- WO-A2-2005/103712
- G. C. MELENDEZ ET AL: "Substance P induces adverse myocardial remodelling via a mechanism involving cardiac mast cells", CARDIOVASCULAR RESEARCH, vol. 92, no. 3, 9 September 2011 (2011-09-09), pages 420-429, XP055103403, ISSN: 0008-6363, DOI: 10.1093/cvr/cvr244
- S. VALDEMARSSON ET AL: "Increased plasma level of substance P in patients with severe congestive heart failure treated with ACE inhibitors", JOURNAL OF INTERNAL MEDICINE, vol. 230, no. 4, 1 October 1991 (1991-10-01), pages 325-331, XP055103405, ISSN: 0954-6820, DOI: 10.1111/j.1365-2796.1991.tb00452.x

## Description

Subject of the present invention is a method for predicting the risk of getting a major adverse cardiac event or dying in a subject that has suffered an acute myocardial infarction comprising:
- determining the level of Protachykinin according to SEQ ID NO. 1, SEQ ID NO. 8, SEQ ID NO. 1 0, SEQ ID NO. 11 or fragments thereof or Protachykinin comprising peptides in a bodily fluid obtained from said subject; and
correlating said level of Protachykinin or fragments thereof or Protachykinin comprising peptides with the risk for getting a major adverse cardiac event or dying, wherein an elevated level is predictive for an enhanced risk of getting a major adverse cardiac event or dying,
wherein fragments of Protachykinin are selected from the group comprising SEQ ID NO. 2, SEQ ID NO. 12, and SEQ ID NO. 13, and wherein said bodily fluid is selected from the group comprising blood, serum, and plasma.

The term "elevated level" means a level above a certain threshold level.

Substance P (SP) is a neuropeptide: an undecapeptide that functions as a neurotransmitter and as a neuromodulator. It belongs to the tachykinin neuropeptide family. Substance P and its closely related neuropeptide neurokinin A (NKA) are produced from a polyprotein precursor after differential splicing of the prePro-Tachykinin A gene. In the CNS, Substance P participates in the pain transmission system.

SP plays a role in nociception, inflammation, plasma extravasation, platelet and leukocyte aggregation in post-capillary venules, and leukocyte chemotactic migration through vessel walls (Otsuka M, Yoshioka K. Neurotransmitter functions of mammalian tachykinins. Physiol Rev. 1993 Apr; 73(2):229-308.)

Neurokinin (NK) receptors are mainly present in coronary vessels and intracardiac ganglia, and not on ventricular or atrial myocardium (Hoover DB, Chang Y, Hancock JC, Zhang L. Actions of tachykinins within the heart and their relevance to cardiovascular disease. Jpn J Pharmacol. 2000 Dec; 84(4):367-73.). A direct action on the NK1 receptor in coronary arteries may cause NO-mediated vasodilatation (Otsuka M, Yoshioka K. Neurotransmitter functions of mammalian tachykinins. Physiol Rev. 1993 Apr;73(2):229-308.), although this effect may be impaired in patients with coronary artery disease (Bossaller C, Habib GB, Yamamoto H, Williams C, Wells S, Henry PD. Impaired muscarinic endothelium-dependent relaxation and cyclic guanosine 5'-monophosphate formation in atherosclerotic human coronary artery and rabbit aorta. J Clin Invest. 1987 Jan; 79(1):170-4.), leading to a dominant NK2 mediated vasconstriction. SP and neurokinin A are negatively inotropic and chronotropic, acting via cholinergic neurons (Hoover DB, Chang Y, Hancock JC, Zhang L. Actions of tachykinins within the heart and their relevance to cardiovascular disease. Jpn J Pharmacol. 2000 Dec;84(4):367-73.). In contrast, NK1 antagonists improve inotropy and lusitropy in rat myocardial infarction (AMI) models whilst SP attenuates the positive inotropic effect of norepinephrine (Wang LL, Guo Z, Han Y, Wang PF, Zhang RL, Zhao YL, Zhao FP, Zhao XY. Implication of Substance P in myocardial contractile function during ischemia in rats. Regul Pept. 2011 Apr 11;167(2-3):185-91.). SP (via the NK1 receptor) has been implicated in myocardial stunning post-AMI in guinea pigs (Chiao H, Caldwell RW. The role of substance P in myocardial dysfunction during ischemia and reperfusion. Naunyn Schmiedebergs Arch Pharmacol. 1996 Mar; 353(4):400-7.).

However, some effects of SP may potentially be beneficial in AMI. SP has been implicated in ischemia post-conditioning (Ren JY, Song JX, Lu MY, Chen H. Cardioprotection by ischemic postconditioning is lost in isolated perfused heart from diabetic rats: Involvement of transient receptor potential vanilloid 1, calcitonin gene-related peptide and substance P. Regul Pept. 2011 Aug 8;169(1-3):49-57.), and NK1 antagonists may lead to a trend in increased AMI size in animal models (Zhang RL, Guo Z, Wang LL, Wu J. Degeneration of capsaicin sensitive sensory nerves enhances myocardial injury in acute myocardial infarction in rats. Int J Cardiol. 2012 Sep 20;160(1):41-7.). Nociceptive perception during ischemia may lead to mobilisation of NK1 receptor expressing bone marrow progenitor cells which may play a role in angiogenesis in the ischemic area (Amadesi S, Reni C, Katare R, Meloni M, Oikawa A, Beltrami AP, Avolio E, Cesselli D, Fortunato O, Spinetti G, Ascione R, Cangiano E, Valgimigli M, Hunt SP, Emanueli C, Madeddu P. Role for substance p-based nociceptive signaling in progenitor cell activation and angiogenesis during ischemia in mice and in human subjects. Circulation. 2012 Apr 10;125 (14):1774-86, S1-19.).

Myocardial (Morrey C, Brazin J, Seyedi N, Corti F, Silver RB, Levi R. Interaction between sensory C-fibers and cardiac mast cells in ischemia/reperfusion: activation of a local renin-angiotensin system culminating in severe arrhythmic dysfunction. 1. J Pharmacol Exp Ther. 2010 Oct;335(1):76-84.) and pulmonary (Guo Z, Wang XP, Wang JP, Zhou RH, Wang LL, Wu J. Up-regulation of substance P in the lungs during acute myocardial ischemia and infarction in rats. Regul Pept. 2010 Feb 25; 160(1-3):160-7.) SP has been observed to be increased in animal models of AMI suggesting a role in AMI.

It has been shown that substance P, through stimulation of the NK-1 R, has the ability to reduce TNF-α-induced apoptosis of human tenocytes Br J Sports Med doi:10.1136/bjsports-2013-092438

Investigations in man have been hampered by the very short half life of SP (12 min) (Conlon, J.M., Sheehan, L. Conversion of substance P to C-terminal fragments in human plasma. Regul. Pept. 1983; 7:335-345.). The recent development of an assay for stable PTA (N-terminal pro-substance P; previously termed also N-terminal Protachikinin A or NT-PTA) which is a surrogate for labile SP (Ernst, A., Suhr, J., Kohrle, J., Bergmann, A. Detection of stable N-terminal protachykinin A immunoreactivity in human plasma and cerebrospinal fluid. Peptides 2008; 29 : 1201-1206.), has enabled studies on the role of this tachykinin system in human disease. Protachykinin fragment PTA 1-37 may be a surrogate marker for Substance P.

WO 2010/128071 describes the stratification of a subject having an acute or chronic disease, like post-myocardial infarction patients, into responder and non-responder to medication like ACE inhibitors, diuretics and beta-blockers and those who have an adverse effect when taking medication. As biomarker vasoactive hormones are used that effect or are correlated with endothelial function/dysfunction. At low levels of vasoactive hormones, the administration of the medication (except for thrombolytic drugs when the hormone is proANP) has an unfavourable effect on a patient having suffered a myocardial infarction, while at higher levels the administration of drugs has a positive effect on a patient having suffered a myocardial infarction. This means according to WO 2010/128071 lower levels of biomarker (with the exception of proANP) indicate a higher risk for the patient as the administration of the medication (except for thrombolytic drugs when the hormone is proANP) has an unfavourable effect on a patient having suffered a myocardial infarction. And further according to WO 2010/128071 higher levels of biomarker (with the exception of proANP) indicate a lower risk for the patient as the administration of the medication (except for thrombolytic drugs when the hormone is proANP) has a positive effect on a patient having suffered a myocardial infarction.

Efficient treatment and management of patients suffering from cardiologic diseases requires a risk stratification system. Tools have been described (NTproBNP, risk scores), but they are imperfect. Thus, there is a need for alternative or additional tools to improve current risk stratification techniques.

Subject of the present invention is a method for predicting the risk of getting a major adverse cardiac event or death in a subject that has suffered an acute myocardial infarction (AMI) comprising:
- determining the level of Protachykinin according to SEQ ID NO. 1, SEQ ID NO. 8, SEQ ID NO. 10, SEQ ID NO. 11 or fragments thereof in a bodily fluid obtained from said subject; and
- correlating said level of Protachykinin SEQ ID NO. 1, SEQ ID NO. 8, SEQ ID NO. 10, SEQ ID NO. 11 or fragments thereof with the risk for getting a major adverse cardiac event or death, wherein an elevated level is predictive for an enhanced risk of getting a major adverse cardiac event or death,
wherein fragments of Protachykinin are selected from the group comprising SEQ ID NO. 2, SEQ ID NO. 12, and SEQ ID NO. 13, and
wherein said bodily fluid is selected from the group comprising blood, serum, and plasma.

In a preferred embodiment subject matter of the present invention is a method for predicting the risk of getting a major adverse cardiac event or death in a subject that has suffered an acute myocardial infarction comprising:
- determining the level of Protachykinin or fragments thereof or Protachykinin comprising peptides in a bodily fluid obtained from said subject, wherein at least one binder is used that binds to PTA 1-37, SEQ ID NO. 2, EEIGANDDLNYWSDWYDSDQIKEELPEPFEHLLQRIA and wherein said binder has an affinity to PTA 1-37 of at least 10⁷ M⁻¹; and
- correlating said level of Protachykinin or fragments thereof or Protachykinin comprising peptides with the a risk for getting a major adverse cardiac event or death, wherein an elevated level is predictive for an enhanced risk of getting a major adverse cardiac event or death.
- Thus, higher level of Protachykinin or fragments thereof or Protachykinin comprising peptides, or higher level of fragments that are determined wherein at least one binder is used that binds to PTA 1-37, SEQ ID NO. 2, indicate a higher risk of getting a major adverse cardiac event or death.

In a specific embodiment this prediction according to the present invention is not the prediction whether said subject shows an unfavourable effect that is caused by a medication or whether said subject is a responder or non-responder to medication. In one specific embodiment the prediction according to the present invention is not the prediction whether said subject shows an unfavourable effect that is caused by a thrombolytic drug or whether said subject is a responder or non-responder to a thrombolytic drug.

Thus, the subject matter of the invention excludes in one specific embodiment the stratification of subjects into responder to a medication, non-responder to a medication and subjects showing an unfavourable effect after having received said medication. In a particular embodiment said medication is a thrombolytic drug. The method of getting a major adverse cardiac event or death in a subject that has suffered an acute myocardial infarction (AMI) according to the present invention is independent of medication in one embodiment of the invention, in particular independent of effects of thrombolytic drugs.

Risk prediction of getting a major adverse cardiac event or death is not the same as diagnosis of a disease or condition or event. Risk prediction means that the biomarker predicts or determines a risk of getting an event or condition that will occur in the future and is not present at the time of measurement and prediction, wherein diagnosis means the determination of a present event or present condition that has occurred. There are many biomarkers that may be suited as diagnostic marker but do not have any predictive power as they change only if the event has occurred. There are also many predictive markers that do not have any diagnostic power. It is in any case not predictable whether a biomolecule may exhibit any diagnostic or predictive power.

In a specific embodiment of the invention said major adverse cardiac event is an acute major adverse cardiac event selected from the group comprising myocardial infarction, stroke, acute heart failure and death. In one specific embodiment the risk for getting said acute adverse event within the next 6 months or 2 years is predicted. In one specific embodiment said AMI has occurred within the last 24 hours before taking the sample bodily fluid from said patient. In one specific embodiment said AMI has occurred within the last week before taking the sample bodily fluid from said patient. In one specific embodiment said AMI has occurred within the last month before taking the sample bodily fluid from said patient. In one specific embodiment said AMI has occurred within the last 2 months before taking the sample bodily fluid from said patient.

In one specific embodiment the subject does not have heart failure at the time the bodily fluid is taken and/or at the time the level of Protachykinin or fragments thereof or Protachykinin comprising peptides are determined in a bodily fluid obtained from said subject.

The primary composite endpoint according to the present invention is major adverse cardiac events (MACE) including all-cause mortality, heart failure (HF) hospitalisation or recurrent AMI (re-AMI), which were evaluated within 2 years. This means that in one embodiment the major adverse cardiac events is selected from the group comprising all-cause mortality, heart failure (HF) hospitalisation or recurrent AMI (re-AMI). In one specific embodiment the major adverse cardiac events is selected from the group comprising all-cause mortality, heart failure (HF) hospitalisation or recurrent AMI (re-AMI) within 2 years.

Hospitalization for HF was defined as a hospital readmission for which HF was the primary reason requiring treatment with high dose diuretics, inotropes or intravenous nitrate. Recurrent AMI was diagnosed using the universal definition (Thygesen K, Alpert JS, White HD; Joint ESC/ACCF/AHA/WHF Task Force for the Redefinition of Myocardial Infarction. Universal definition of myocardial infarction. Circulation 2007;116:2634-53.). Secondary endpoints were composites of death and/or re-AMI and death and/or HF readmission, and re-AMI individually. The endpoint of death and/or re-AMI at 6 months was used in analyses involving the GRACE score as this time-point was used in development of the risk score. Endpoints were obtained by reviewing the local hospital databases and the Office of National Statistics Registry and phone calls to patients. This means that in one embodiment the major adverse cardiac events is selected from the group comprising composites of death and/or re-AMI and death and/or HF readmission, and re-AMI individually. In one specific embodiment the major adverse cardiac events is selected from the group comprising composites of death and/or re-AMI and death and/or HF readmission, and re-AMI individually within six months.

In one specific embodiment the major adverse cardiac events is death and/or re-AMI. In one specific embodiment the major adverse cardiac events is death and/or re-AMI at 6 months.

Protachykinin and fragments thereof or Protachykinin comprising peptides may be used as a surrogate marker for released Substance P and/or for released neurokinin as Substance P / neurokinin and Protachykinin and fragments thereof or Protachykinin comprising peptides are released in equimolar amounts from Protachykinin. In one embodiment the level of Protachykinin and fragments thereof or Protachykinin comprising peptides excluding Substance P or neurokinin is determined. In another embodiment the level of Substance P or neurokinin is determined. The level of a certain peptide or peptide fragment may be determined by any available analytical method e.g. be using at least one binder the binds to said Protachykinin and fragments thereof or Protachykinin comprising peptides. Such a binder may be an antibody. Said level may be determined by e.g. mass spectroscopy.

In one embodiment of the above methods an increased level of Pro-Tachykinin, its splice variants or fragments thereof is a level above a threshold wherein said threshold is (about) 100 pmol/l or above 100 pmol/l, preferably (about) 80 pmol/L or above 80 pmol/L, preferably (about) 70 pmol/L or above 70 pmol/L.

Thresholds have to be seen in light of the calibration method used and the above values have to be seen in light of the assays and calibration methods used in the present examples 1, 2 and 3. With the calibration method used in the present invention according to Example 1, the median level of Pro-Tachykinin in healthy subjects has been determined at 53 pmol/L.

In one embodiment of the invention said major adverse cardiac event is an acute major adverse cardiac event selected from the group comprising myocardial infarction, stroke, acute heart failure.

In one embodiment of the invention said sample of bodily fluid from said subject has been taken within a certain time frame after the AMI has occurred, this time frame being 2 months, more preferably 1 month, more preferably 1 week, most preferably within 24 hours.

In one embodiment of the invention said elevated level is predictive for an enhanced risk of getting a major adverse cardiac event or death within the next 6 months or within the next 2 years.

Throughout the specification the term Pro-Tachykinin and Pro-Tachykinin A (PTA) are used synonymously. The term includes all splice variants of Pro-Tachykinin A, namely αPTA, βPTA, γPTA, and δPTA.

The term "determining the level of Pro-Tachykinin, its splice variants or fragments thereof means that usually the immunoreactivity towards a region within the before mentioned molecules is determined. This means that it is not necessary that a certain fragment is measured selectively. It is understood that a binder which is used for the determination of the level of Pro-Tachykinin or fragments thereof binds to a fragment that comprises the region of binding of said binder wherein fragments of Protachykinin are selected from the group comprising
SEQ ID NO. 2, SEQ ID NO. 12, and SEQ ID NO. 13. Said binder may be an antibody or antibody fragment or a non-IgG Scaffold.

This means throughout the specification and the claims the term "determining the level of Protachykinin or fragments thereof or Protachykinin comprising peptides" is equivalent with "determining the immunoreactivity towards a region within the before mentioned molecules" and is equivalent with "determining the level of a binder e.g. antibody that binds to a region that is comprised in Protachykinin or fragments thereof or Protachykinin comprising peptides".

The term "subject" as used herein refers to a living human or non-human organism. Preferably herein the subject is a human subject.

The correlation between the level of Protachykinin or Protachykinin comprising peptides in a bodily fluid obtained from said subject and the risk of getting a major adverse cardiac event is continuous, i.e. the higher the level the higher the risk. This can be seen from the data e.g. in Table 2. In comparison to the first quartile the second, third and fourth quartile exhibits higher risks respectively.

For the sake of practicability the person skilled in the art may use threshold(s).

Thus, the term "elevated level" may mean a level above a threshold level.

A bodily fluid may be selected from the group comprising blood, serum, and plasma.

In a specific embodiment said cardiovascular disease at the time the sample of bodily fluid is taken from said subject is acute myocardial infarction (AMI). In one specific embodiment said AMI is a STEMI (ST-segment elevation myocardial infarction) or a NSTEMI (non-ST-segment elevation myocardial infarction). In one embodiment of the invention said subject had an AMI wherein in one embodiment said patient had a cardiac troponin level above the 99^{th} percentile with at least one of the following: chest pain lasting > 20 minutes or diagnostic serial electrocardiographic changes consisting of new pathological Q waves or ST-segment and T-wave changes.

In another specific embodiment of the invention the prediction of an adverse event in a subject or the identification of a subject having an enhanced risk for getting another adverse event after having had an AMI is improved by additionally determining and using the level of at least one further marker selected from the group comprising: Troponin I, Troponin T, CRP, LpLA2, Cystatin C and natriuretic peptides of the A- and the B-type as well as their precursors and fragments thereof including ANP, proANP, NT-proANP, MR-proANP, BNP, proBNP, NT-proBNP triglycerides, HDL cholesterol or subfractions thereof, LDL cholesterol or subfractions thereof, GDF15, ST2, copeptin, and any score, such as for instance the PURSUIT, TIMI, GRACE and FRISC risk score (B.E Backus, A.J Six, J.H Kelder, W.B Gibler, F.L Moll,1 and P.A Doevendans, Risk Scores for Patients with Chest Pain: Evaluation in the Emergency Department, Curr Cardiol Rev. 2011 February; 7(1): 2-8.).

In a very specific embodiment of the method according to the invention in addition to the level of Protachykinin or fragments thereof, wherein fragments of Protachykinin are selected from the group comprising SEQ ID NO. 2, SEQ ID NO. 12, and SEQ ID NO. 13, or Protachykinin comprising peptides the level of the following marker is determined and used: proBNP or fragments or precursors thereof having at least 12 amino acids and/or CRP.

In another specific embodiment of the invention additionally at least one clinical parameter is determined selected from the group comprising: age, systolic blood pressure, diastolic blood pressure, antihypertensive treatment, body mass index, presence of diabetes mellitus, current smoking.

Pro-Tachykinin may have the following sequence(s):
SEQ ID NO. 1 (Pro-Tachykinin A (1-107)

EEIGANDDLNYWSDWYDSDQIKEELPEPFEHLLQRIARRPKPQQFFGLMGKRDADSSI
EKQVALLKALYGHGQISHKRHKTDSFVGLMGKRALNSVAYERSAMQNYERRR

Fragments of Pro-Tachykinin that may be determined in a bodily fluid may be *e.g.* selected from the group of the following fragments:
SEQ ID NO. 2 (Pro-Tachykinin 1-37, P37)
   EEIGANDDLNYWSDWYDSDQIKEELPEPFEHLLQRIA
SEQ ID NO. 3 (Substance P), which is not part of the invention,
   RPKPQQFFGLM(-NH2)
SEQ ID NO. 4 (Neuropeptide K), which is not part of the invention,
   DADSSIEKQVALLKALYGHGQISHKRHKTDSFVGLM (-NH2)
SEQ ID NO. 5 (Neuropeptide Gamma), which is not part of the invention,
   GHGQISHKRHKTDSFVGLM (-NH2)
SEQ ID NO. 6 (Neurokinin 1), which is not part of the invention,
   HKTDSFVGLM(-NH2)
SEQ ID NO. 7 (C-terminal flanking peptide, PTA 1 92-107), which is not part of the invention,
   ALNSVAYERSAMQNYE
SEQ ID NO. 8 (PTA Isoform alpha) 
EEIGANDDLNYWSDWYDSDQIKEELPEPFEHLLQRIARRPKPQQFFGLMGKRDADSSI
EKQVALLKALYGHGQISHKMAYERSAMQNYERRR
SEQ ID NO. 9 (PTA Isoform beta) 
EEIGANDDLNYWSDWYDSDQIKEELPEPFEHLLQRIARRPKPQQFFGLMGKRDADSSI
EKQVALLKALYGHGQISHKRHKTDSFVGLMGKRALNSVAYERSAMQNYERRR
SEQ ID NO. 10 (PTA Isoform delta) 
EEIGANDDLNYWSDWYDSDQIKEELPEPFEHLLQRIARRPKPQQFFGLMGKRDAGHG
QISHKMAYERSAMQNYERRR
SEQ ID NO. 11 (PTA Isoform gamma) 
EEIGANDDLNYWSDWYDSDQIKEELPEPFEHLLQRIARRPKPQQFFGLMGKRDAGHG
QISHKRHKTDSFVGLMGKRALNSVAYERSAMQNYERRRSEQ
SEQ ID NO. 12 (PTA3-22)
   GANDDLNYWSDWYDSDQIK
SEQ ID NO. 13 (PTA 21-36)
   IKEELPEPFEHLLQRI

Determining the level of PTA, its splice variants or fragments thereof may mean that the immunoreactivity towards PTA or fragments thereof, wherein fragments of Protachykinin are selected from the group comprising SEQ ID NO. 2, SEQ ID NO. 12, and SEQ ID NO. 13, is determined. A binder used for determination of PTA, its splice variants or fragments thereof depending of the region of binding may bind to more than one of the above displayed molecules. This is clear to a person skilled in the art.

In a more specific embodiment of the method according to the present invention the level of P37 (PTA 1-37, SEQ ID NO. 2, EEIGANDDLNYWSDWYDSDQIKEELPEPFEHLLQRIA) is determined. In an even more specific embodiment according to the present invention at least one or two binders are used that bind to PTA 1-37, SEQ ID NO. 2, EEIGANDDLNYWSDWYDSDQIKEELPEPFEHLLQRIA, in case of more than one binder they bind preferably to two different regions within PTA 1-37, SEQ ID NO. 2, EEIGANDDLNYWSDWYDSDQIKEELPEPFEHLLQRIA. Said binder(s) may preferably be an antibody or a binding fragment thereof.

In an even more specific embodiment binder(s) are used for the determination of PTA its variants and fragments that bind to one or both, respectively, of the following regions within PTA 1-37:

| | |
|---|---|
| GANDDLNYWSDWYDSDQIK | PTA 3-22 (SEQ ID NO. 12) |
| IKEELPEPFEHLLQRI | PTA 21-36 (SEQ ID NO. 13) |

In a specific embodiment the level of PTA, its splice variants or fragments thereof are measured with an immunoassay using antibodies or fragments of antibodies binding to PTA, its splice variants or fragments thereof. An immunoassay that may be useful for determining the level of PTA, its splice variants or fragments thereof may comprise the steps as outlined in Example 1. All thresholds and values have to be seen in correlation to the test and the calibration used according to Example 1. A person skilled in the art may know that the absolute value of a threshold might be influenced by the calibration used. This means that all values and thresholds given herein are to be understood in context of the calibration used in herein (Example 1).

In a more specific embodiment of the method according to the present invention the level of Protachykinin is determined.

According to the invention the diagnostic binder to PTA or the other additional biomarkers is selected from the group consisting of antibodies *e.g.* IgG, a typical full-length immunoglobulin, or antibody fragments containing at least the F-variable domain of heavy and/or light chain as *e.g*. chemically coupled antibodies (fragment antigen binding) including but not limited to Fab-fragments including Fab minibodies, single chain Fab antibody, monovalent Fab antibody with epitope tags, *e.g*. Fab-V5Sx2; bivalent Fab (mini-antibody) dimerized with the CH3 domain; bivalent Fab or multivalent Fab, *e.g.* formed via multimerization with the aid of a heterologous domain, *e.g.* via dimerization of dHLX domains, *e.g.* Fab-dHLX-FSx2; F(ab')2-fragments, scFv-fragments, multimerized multivalent or/and multispecific scFv-fragments, bivalent and/or bispecific diabodies, BITE® (bispecific T-cell engager), trifunctional antibodies, polyvalent antibodies, e.g. from a different class than G; single-domain antibodies, e.g. nanobodies derived from camelid or fish immunoglobulines. In a specific embodiment the level of PTA, its splice variants or fragments thereof or the other additional biomarkers is measured with an assay using binders selected from the group comprising aptamers, non-Ig scaffolds as described in greater detail below binding to PTA, its splice variants or fragments thereof or alternatively to the additional biomarkers.

Binder that may be used for determining the level of Protachykinin (PTA,) its splice variants or fragments thereof exhibit an affinity constant to PTA, its splice variants or fragments thereof of at least 10⁷ M⁻¹, preferred 10⁸ M⁻¹, preferred affinity constant is greater than 10⁹ M⁻¹, most preferred greater than 10¹⁰ M⁻¹. A person skilled in the art knows that it may be considered to compensate lower affinity by applying a higher dose of compounds and this measure would not lead out-of-the-scope of the invention. Binding affinity may be determined using the Biacore method, offered as service analysis *e.g.* at Biaffin, Kassel, Germany (http://www.biaffin.com/de/), see also above.

Binder that may be used for determining the level of PTA, its splice variants or fragments thereof exhibit an affinity constant to PTA, its splice variants or fragments thereof of at least 10⁷ M⁻¹, preferred 10⁸ M⁻¹, preferred affinity constant is greater than 10⁹ M⁻¹, most preferred greater than 10¹⁰ M⁻¹. A person skilled in the art knows that it may be considered to compensate lower affinity by applying a higher dose of compounds and this measure would not lead out-of-the-scope of the invention. Binding affinity may be determined using the Biacore method, offered as service analysis *e.g.* at Biaffin, Kassel, Germany (http://www.biaffin.com/de/).

### Affinty Constants

To determine the affinity of the antibodies, the kinetics of binding of PTA, its splice variants or fragments thereof to immobilized antibody was determined by means of label-free surface plasmon resonance using a Biacore 2000 system (GE Healthcare Europe GmbH, Freiburg, Germany). Reversible immobilization of the antibodies was performed using an anti-mouse Fc antibody covalently coupled in high density to a CM5 sensor surface according to the manufacturer's instructions (mouse antibody capture kit; GE Healthcare). (Lorenz et al.," Functional Antibodies Targeting IsaA of Staphylococcus aureus Augment Host Immune Response and Open New Perspectives for Antibacterial Therapy"; Antimicrob Agents Chemother. 2011 January; 55(1): 165-173.)

A human PTA-control sample is available by ICI-Diagnostics, Berlin, Germany http://www.ici-diagnostics.com/. The assay may also be calibrated by synthetic (for our experiments we used synthetic P37, SEQ ID NO. 2) or recombinant PTA, its splice variants or fragments thereof.

In one embodiment of the invention said method is performed more than once in order to monitor the risk of getting a major adverse cardiac event in a subject or in order to monitor the course of treatment. In one specific embodiment said monitoring is performed in order to evaluate the response of said subject to preventive and/or therapeutic measures taken.

In one embodiment of the invention the method is used in order to stratify said subjects into risk groups.

Subject matter of the invention is further an assay, referred to in the claims as "device", for determining PTA, its splice variants or fragments in a sample comprising two binders that bind to two different regions within the region of PTA that is amino acid 3-22 (GANDDLNYWSDWYDSDQIK, SEQ ID NO. 12) and amino acid 21-36 (IKEELPEPFEHLLQRI, SEQ ID NO. 13) wherein each of said regions comprises at least 4 or 5 amino acids.

In one embodiment of the assays for determining PTA, its splice variants or fragments in a sample according to the present invention the analytical assay sensitivity of said assay is able to quantify the PTA, its splice variants or PTA fragments of healthy subjects and is < 20 pmol/, preferably < 10 pmol/l and more preferably < 5 pmol/l.

In one embodiment of the assays for determining PTA, its splice variants or fragments in a sample according to the present invention such assay is a sandwich assay, preferably a fully automated assay. It may be an ELISA, a fully automated assay or a manual assay. It may be a so-called POC-test (point -of-care). Examples of automated or fully automated assay comprise assays that may be used for one of the following systems: Roche Elecsys®, Abbott Architect®, Siemens Centauer®, Brahms Kryptor®, Biomerieux Vidas®, Alere Triage®. Examples of test formats are provided above.

In one embodiment of the assays for determining PTA, its splice variants or fragments in a sample according to the present invention at least one of said two binders is labelled in order to be detected. Examples of labels are provided above.

In one embodiment of the assays for determining PTA, its splice variants or fragments in a sample according to the present invention at least one of said two binders is bound to a solid phase. Examples of solid phases are magnetic beads, polystyrene tubes or microtiter plates. In one embodiment a homogenous assay is used, i.e. using Time Resolved Amplified Cryptate Emission (TRACE) technologies.

In one embodiment of the assays for determining PTA, its splice variants or fragments in a sample according to the present invention said label is selected from the group comprising chemiluminescent label, enzyme label, fluorescence label, radioiodine label.

A further subject of the present invention is a kit comprising an assay according to the present invention wherein the components of said assay may be comprised in one or more container.

In a specific embodiment of the method according to the invention additionally at least one clinical parameter is determined selected from the group comprising age, gender, systolic blood pressure, diastolic blood pressure, antihypertensive treatment (AHT), body mass index, waist circumference, waist-hip-ratio, current smoker, diabetes heredity and previous cardiovascular disease (CVD).

Subject matter of the present invention is further a method for predicting the risk of getting a major adverse cardiac event in a subject suffering from AMI or identifying a subject suffering from AMI having an enhanced risk for getting a major adverse cardiac event according to the invention, wherein the level of Protachykinin or fragments thereof of at least 5 amino acids either alone or in conjunction with other prognostically useful laboratory or clinical parameters is used for the prediction of a subject's risk for getting a major adverse cardiac event by a method which may be selected from the following alternatives:
- Comparison with the median of the level of Protachykinin or fragments thereof or Protachykinin comprising peptides in an ensemble of pre-determined samples in a population of "healthy" or "apparently healthy" subjects,
- Comparison with a quantile of the level of Protachykinin or fragments thereof or Protachykinin comprising peptides in an ensemble of pre-determined samples in a population of "healthy" or "apparently healthy" subjects,
- Calculation based on Cox Proportional Hazards analysis or by using Risk index calculations such as the NRI (Net Reclassification Index) or the IDI (Integrated Discrimination Index).

In one embodiment of the method according to the invention said a method is performed more than once in order to monitor the risk of getting a major adverse cardiac event in a subject suffering from AMI.

In another embodiment of the method according to the invention said monitoring is performed in order to evaluate the response of said subject suffering from AMI to preventive and/or therapeutic measures taken.

In another embodiment of the method according to the in order invention the method is used to stratify said subjects suffering from AMI into risk groups.

Also encompassed by the present invention is a point-of-care device for performing a method according to the invention.

Also encompassed by the present invention is an assay and/or kit for performing a method according to the invention.

### FIGURE DESCRIPTION

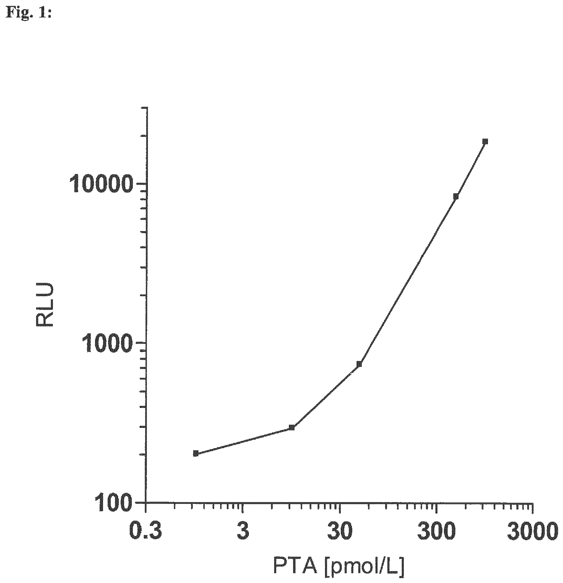

**Figure 1****:** shows a typical PTA dose/ signal curve. Standard curve PTA.
**Figure 2****:** Profile of plasma PTA over 5 days following AMI, in those with (in red) or without (in green) MACE at 2 years.
**Figure 3****:** Reclassification plot showing up and down reclassification of the probabilities of events in survivors and patients with the endpoint of death and/or MI at 6 months.
**Figure 4****:** Classification tree for the endpoint of death and/or MI at 6 months.
**Figure 5****:** Classification trees using PTA as the initial classifier, for the endpoint of death and/or MI at 6 months.
**Figure 6**: Kaplan Meier plot showing death and/or MI events with time stratified according to plasma PTA below or above 72.1 pmol/L. Survival Functions

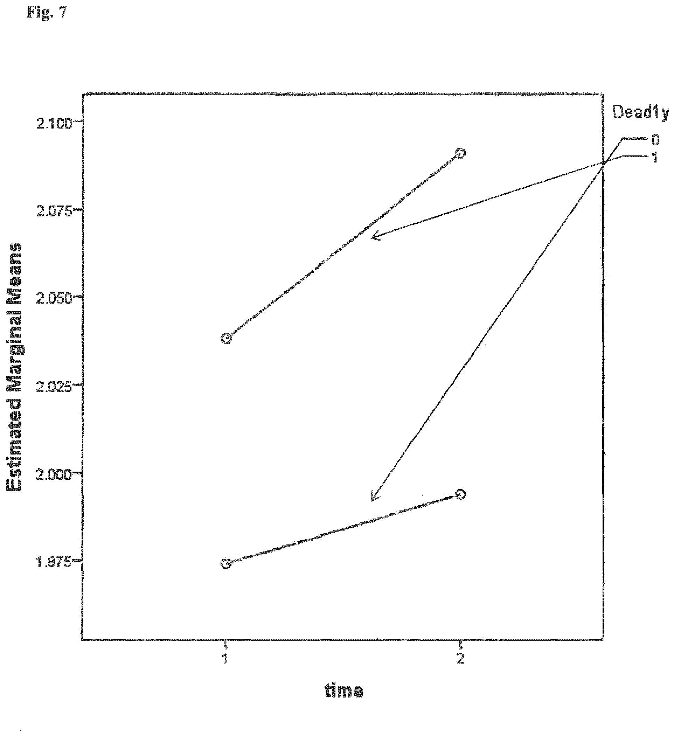

**Figure 7****:** Estimated Marginal Means of MEASURE_1

### Examples

### Example 1

### PTA-immunoassay

### Development of anti PTA Antibodies

### Peptides/ conjugates for Immunization:

Peptides for immunization were synthesized (JPT Technologies, Berlin, Germany) with an additional N-terminal Cystein residue for conjugation of the peptides to bovine serum albumin (BSA). The peptides were covalently linked to BSA by using Sulfo-SMCC (Perbio-science, Bonn, Germany). The coupling procedure was performed according to the manual of Perbio.

**Table 1:**

| Peptide for immunization | PTA Sequence |
|---|---|
| (C)GANDDLNYWSDWYDSDQIK | 3-22 (SEQ ID NO. 12) |
| (C) IKEELPEPFEHLLQRI | 21-36 (SEQ ID NO. 13) |

The antibodies were generated according to the following method:
A BALB/c mouse was immunized with 100 µg peptide-BSA-conjugate at day 0 and 14 (emulsified in 100 µl complete Freund's adjuvant) and 50 µg at day 21 and 28 (in 100 µl incomplete Freund's adjuvant). Three days before the fusion experiment was performed, the animal received 50 µg of the conjugate dissolved in 100 µl saline, given as one intraperitonal and one intravenous injection.

Splenocytes from the immunized mouse and cells of the myeloma cell line SP2/0 were fused with 1 ml 50 % polyethylene glycol for 30 s at 37 °C. After washing, the cells were seeded in 96-well cell culture plates. Hybrid clones were selected by growing in HAT medium [RPMI 1640 culture medium supplemented with 20% fetal calf serum and HAT-supplement]. After two weeks the HAT medium is replaced with HT Medium for three passages followed by returning to the normal cell culture medium.

The cell culture supernatants were primary screened for antigen specific IgG antibodies three weeks after fusion. The positive tested microcultures were transferred into 24-well plates for propagation. After retesting the selected cultures were cloned and recloned using the limiting-dilution technique and the isotypes were determined.

(Lane, R.D. "A short-duration polyethylene glycol fusiontechnique for increasing production of monoclonal antibody-secreting hybridomas", J. Immunol. Meth. 81: 223-228; (1985), Ziegler, B. et al. "Glutamate decarboxylase (GAD) is not detectable on the surface of rat islet cells examined by cytofluorometry and complement-dependent antibody-mediated cytotoxicity of monoclonal GAD antibodies", Horm. Metab. Res. 28: 11-15, (1996)).

### Monoclonal antibody production

Antibodies were produced via standard antibody production methods (Marx et al., Monoclonal Antibody Production (1997), ATLA 25, 121) and purified via Protein A-chromatography. The antibody purities were > 95 % based on SDS gel electrophoresis analysis.

### Labelling and coating of antibodies:

All antibodies were labelled with acridinium ester according the following procedure:
Labelled compound (tracer, anti PTA 3-22): 100 µg (100 µl) antibody (1 mg/ml in PBS, pH 7.4, was mixed with 10 µl Acridinium NHS-ester (1 mg/ml in acetonitrile, InVent GmbH, Germany) (EP 0353971) and incubated for 20 min at room temperature. Labelled antibody was purified by gel-filtration HPLC on Bio-Sil SEC 400-5 (Bio-Rad Laboratories, Inc., USA) The purified labelled antibody was diluted in (300 mmol/l potassiumphosphate, 100 mmol/l NaCl, 10 mmol/l Na-EDTA, 5 g/l bovine serum albumin, pH 7.0). The final concentration was approx. 800.000 relative light units (RLU) of labelled compound (approx. 20 ng labeled antibody) per 200 µl. Acridiniumester chemiluminescence was measured by using an AutoLumat LB 953 (Berthold Technologies GmbH & Co. KG).

Solid phase antibody (coated antibody):
Solid phase: Polystyrene tubes (Greiner Bio-One International AG, Austria) were coated (18 h at room temperature) with anti PTA 22-36 antibody (1.5 µg antibody/0.3 ml 100 mmol/l NaCl, 50 mmol/l Tris/HCl, pH 7.8). After blocking with 5 % bovine serum albumine, the tubes were washed with PBS, pH 7.4 and vacuum dried.

### PTA Immunoassay:

50 µl of sample (or calibrator) was pipetted into coated tubes, after adding labeled antibody (200ul), the tubes were incubated for 2 h at 18-25 °C. Unbound tracer was removed by washing 5 times (each 1 ml) with washing solution (20 mmol/l PBS, pH 7.4, 0.1 % Triton X-100). Tube-bound labelled antibody was measured by using a Luminumeter LB 953, Berthold, Germany.

### Calibration:

The assay was calibrated, using dilutions of synthetic P37, diluted in 20 mM K2PO4, 6 mM EDTA, 0,5% BSA, 50µM Amastatin, 100µM Leupeptin, pH 8.0. PTA control plasma is available at ICI-diagnostics, Berlin, Germany.

The analytical assay sensitivity was (the median signal generated by 20 determinations of 0-calibrator (no addition of PTA) + 2SD2 standard deviations (SD), the corresponding PTA concentration is calculated from a standard curve) 4.4 pmol/L.

### Example 2 - Study in patients with acute myocardial infarction

### Study Population:

We studied 1148 STEMI (= ST-segment Elevation Myocardial Infarction) and NSTEMI (= Non-ST-segment Elevation Myocardial Infarction) patients admitted to University Hospitals of Leicester NHS trust between August 2004 and April 2007. This observational cohort study complied with the Declaration of Helsinki and was approved by the local ethics committee; written informed consent was obtained from patients. AMI was diagnosed if a patient had a cardiac troponin I level above the 99th centile with at least one of the following: chest pain lasting >20 minutes or diagnostic serial electrocardiographic changes consisting of new pathological Q waves or ST-segment and T-wave changes (Thygesen K, Alpert JS, White HD; Joint ESC/ACCF/AHA/WHF Task Force for the Redefinition of Myocardial Infarction. Universal definition of myocardial infarction. Circulation 2007;116:2634-53.). Patients with known malignancy, renal replacement therapy or surgery in the previous month were excluded. Estimated glomerular filtration rate (eGFR) was calculated from the simplified Modification of Diet in Renal Disease formula (Smilde TD, van Veldhuisen DJ, Navis G, Voors AA, Hillege HL. Drawbacks and prognostic value of formulas estimating renal function in patients with chronic heart failure and systolic dysfunction. Circulation 2006;114:1572-80.). All patients received standard medical treatment and revascularisation at the discretion of the attending physician.

### Plasma samples:

Blood samples (anticoagulated with EDTA and aprotinin) were drawn after 15 minutes bed rest, immediately after diagnosis and within 36 h of symptom onset. Plasma was stored at - 80°C until assayed in a single batch for blinded determination of plasma PTA and NTproBNP.

### Echocardiography:

Transthoracic echocardiography was performed in 895 (77.9%) patients during the index admission, using either a Sonos 5500 or IE 33 instrument (Philips Medical Systems, Reigate, UK). A 16-segment left ventricular wall motion index (LVWMI) score was performed based on the American Society of Echocardiography method (Schiller NB, Shah PM, Crawford M, et al. Recommendations for quantitation of the left ventricle by two-dimensional echocardiography. American Society of Echocardiography Committee on Standards, Subcommittee on Quantitation of Two-Dimensional Echocardiograms. J Am SocEchocardiogr. 1989; 2: 358-367.). In suitable patients left ventricular ejection fraction (LVEF) was calculated using the biplane method of discs formula. LV systolic dysfunction (LVSD) was defined as either an LVEF<40% or a LVWMI >1.8.

Global Registry of Acute Coronary Events (GRACE) Scoring: Based on an international observational database of acute coronary syndrome patients, GRACE scores can be calculated on initial presentation to predict in-hospital mortality (Granger CB, Goldberg RJ, Dabbous O, et al. Global Registry of Acute Coronary Events Investigators. Predictors of hospital mortality in the global registry of acute coronary events. Arch Intern Med. 2003; 163 : 2345-53.) or for 6 month major adverse cardiac events (MACE), defined as death and/or re-MI (Eagle KA, Lim MJ, Dabbous OH, et al. A validated prediction model for all forms of acute coronary syndrome: estimating the risk of 6-month post discharge death in an international registry. JAMA 2004;291:2727-33.). We used GRACE scores on discharge for comparison with 6 month death and/or re-AMI.

### End points:

The primary composite endpoint was major adverse cardiac events (MACE) including all-cause mortality, heart failure (HF) hospitalisation or recurrent AMI (re-AMI), which were evaluated within 2 years. Hospitalization for HF was defined as a hospital readmission for which HF was the primary reason requiring treatment with high dose diuretics, inotropes or intravenous nitrate. Recurrent AMI was diagnosed using the universal definition (Thygesen K, Alpert JS, White HD; Joint ESC/ACCF/AHA/WHF Task Force for the Redefinition of Myocardial Infarction. Universal definition of myocardial infarction. Circulation 2007;116:2634-53.). Secondary endpoints were composites of death and/or re-AMI and death and/or HF readmission, and re-AMI individually. The endpoint of death and/or re-AMI at 6 months was used in analyses involving the GRACE score as this time-point was used in development of the risk score. Endpoints were obtained by reviewing the local hospital databases and the Office of National Statistics Registry and phone calls to patients.

We achieved 100% follow-up. Statistical analysis: Statistical analyses were performed on SPSS Version 20 (SPSS Inc, Chicago, Illinois) and Stata 12.1 (Texas,USA). Assuming an event rate of 15% and that the covariates predict up to 30% of the variance of the biomarker, a sample size of 600 patients would be powered (90% at p<0.05) to detect a hazard ratio of the biomarker of 1.5. Biomarker levels were log10 transformed and therefore hazard ratios refer to a tenfold rise in the levels of these markers. GRACE scores were used as the original scores. Non-parametric tests were employed against non-Gaussian data (Mann-Whitney U-test, Kruskal Wallis test and Spearman (rs) correlations). Independent predictors of PTA levels were assessed using univariate general linear models. To assess prognostic value of biomarkers, a 'base' model was generated using Cox survival analysis, which included variables that were significantly (p<0.10) associated with any of the study end points on univariate analysis (age, gender, previous history of ischemic heart disease (IHD), hypertension or diabetes, Killip Class, eGFR, and log troponin I). Biomarkers (NTproBNP, PTA) were added to this base model to evaluate the relative prognostic value of each with all variables entered simultaneously. A second 'comparative' Cox model, was used to assess the relative prognostic power of these biomarkers and the GRACE score. The additional prognostic value of PTA to the GRACE score was evaluated by reclassification analysis with calculation of category-free net reclassification improvement (NRI) as described by Pencina et al (Pencina MJ, D'Agostino RB Sr, Steyerberg EW. Extensions of net reclassification improvement calculations to measure usefulness of new biomarkers. Stat Med. 2011; 30 : 11-21.). We constructed classification trees using Chisquared Automatic Interaction Detection (CHAID, analysis performed using SPSS), which chooses at each step the biomarker that has the strongest interaction with the dependent variable.

### Patient Characteristics

The characteristics of the study population are shown in Table 2, according to PTA quartiles. Patients with higher PTA levels were older, female, with past histories of hypertension, IHD, diabetes, HF, and had higher GRACE scores, NTproBNP and glucose levels. They also had more impaired cardiac and renal function.

**Table 2:**

| | | PTA quartiles | | | | |
|---|---|---|---|---|---|---|
| | All | 1 | 2 | 3 | 4 | P Value |
| | | <52.0 pmol/L | 52.0-65.19 pmol/L | 65.19-89.1 pmol/L | >89.1 pmol/L | |
| | n=1148 | n=288 | n=286 | n=288 | n=286 | |
| PTA (pmol/L) | 77.2 ± 55.7 | 42.2 ± 7.43 | 58.4 ± 4.0 | 75.6 ± 7.1 | 132.9 ± 87.4 | <0.0005 |
| NTproBNP(pmol/L) | 1849 ± 2108 | 891.3 ± 1062 | 1339 ± 1641 | 1874 ±2030 | 3300 ± 2569 | <0.0005 |

| **Demographics** | | | | | | |
|---|---|---|---|---|---|---|
| Age (years) | 66.2 ± 12.8 | 58.3 ± 11.2 | 63.1 ± 11.0 | 68.1 ± 11.9 | 75.4 ± 10.3 | <0.001 |
| Male (%) | 825 (72) | 235 (82) | 214 (75) | 208 (72) | 168 (58) | <0.001 |
| ST elevation MI | 545 (47) | 144 (50) | 132 (46) | 149 (52) | 120 (42) | NS |
| Previous History | | | | | | |
| IHD | 379 (33) | 67 (23) | 80 (28) | 91 (31) | 141 (49) | <0.001 |
| Heart Failure | 46 (4) | 3 (1) | 8 (3) | 10 (3) | 19 (7) | <0.003 |
| Hypertension | 596 (52) | 125 (44) | 134 (47) | 152 (53) | 185 (65) | <0.001 |
| Diabetes Mellitus | 266 (23) | 53 (18) | 71 (25) | 61 (21) | 81 (28) | 0.032 |
| Killip Class>1 | 426 (40) | 61 (24) | 92 (35) | 121 (45) | 152 (56) | <0.001 |
| Glucose (mmol/L) | 8.9 ± 4.2 | 8.5 ± 3.9 | 8.7 ± 3.9 | 8.4 ± 3.5 | 9.9 ± 5.4 | <0.001 |
| Troponin I (ng/mL) | 13.1 ± 25.8 | 13.2 ± 26.7 | 12.0 ± 24.4 | 15.0 ± 27.9 | 12.1 ± 24.2 | NS |
| eGFR (ml/min/1.73m²) | 65.6 ± 20.1 | 77.9 ± 17.7 | 71.4 ± 15.5 | 64.4 ± 16.6 | 48.9 ± 17.9 | <0.001 |

| **Risk Markers on Discharge** | | | | | | |
|---|---|---|---|---|---|---|
| Echocardiographic LVSD [n=893] | | | | | | |
| LV wall motion index | 1.47 ± 0.42 | 1.38 ± 0.37 | 1.46 ± 0.42 | 1.46 ± 0.41 | 1.60 ± 0.43 | <0.001 |
| LV ejection fraction | 42.1 ± 14.5 | 44.8 ± 13.8 | 43.8 ± 14.3 | 41.4 ± 13.8 | 38.3 ± 15.2 | <0.001 |
| GRACE score | 120.0 ± 32.7 | 99.7 ± 26.6 | 109.6 ± 26.9 | 125.6 ± 28.4 | 144.5 ± 29.9 | <0.001 |

| **Treatment** | | | | | | |
|---|---|---|---|---|---|---|
| Aspirin | 963 (84) | 255 (89) | 255(89) | 238 (82) | 215 (75) | <0.001 |
| Beta-blocker | 920 (80) | 256 (89) | 238 (83) | 230 (80) | 196 (69) | <0.001 |
| ACE inhibitor or ARB* | 940 (82) | 249 (87) | 234 (82) | 245 (85) | 212 (74) | <0.001 |
| Statin | 1002 (87) | 270 (94) | 258 (90) | 260 (90) | 214 (75) | <0.001 |
| Loop Diuretic | 289 (25) | 39 (14) | 59 (21) | 69 (24) | 122 (43) | <0.001 |

| **End Points (2 years)** | | | | | | |
|---|---|---|---|---|---|---|
| Major Adverse Cardiac Events | 324 (28) | 45 (16) | 53 (18) | 77 (27) | 149 (52) | <0.001 |
| Death | 140 (12) | 11 (4) | 11 (4) | 31 (11) | 87 (30) | <0.001 |
| Non-fatal major Adverse Cardiac Events | 230 (20) | 41 (14) | 46 (16) | 56 (19) | 87 (30) | <0.001 |
| Heart Failure | 112 (9.8) | 13 (5) | 19 (7) | 28 (10) | 52 (18) | <0.001 |
| Re-AMI | 149 (13) | 29 (10) | 35 (12) | 33 (11) | 52 (18) | <0.021 |

Characteristics of the 1148 AMI patients according to PTA quartiles on admission. Numerical data are presented as n (%). P values are quoted for ANOVA (F statistic) or Kruskal-Wallis tests for continuous or categorical variables respectively. Numbers (%) or Mean± SD are reported.

### Correlation analysis

Spearman analysis (rs) revealed PTA was significantly correlated to age (0.521), eGFR (-0.555), diastolic BP (-0.178), NTproBNP (0.428), ejection fraction (-0.175) (all P<0.0005) and heart rate (0.100, P<0.001). PTA was not correlated to troponin or peak creatine kinase levels.

A univariate general linear model indicated the following independent predictors of PTA level, in descending order according to variance accounted for in the model (Table 3): eGFR, age, past history diabetes and IHD, Killip class above 1, LV wall motion score, female gender and diastolic BP. eGFR accounted for the majority of the variance (20.7%).

**Table 3:**

| Variable | Type III sum of squares | F statistic | P value |
|---|---|---|---|
| | | | |
| eGFR | 2.299 | 107.384 | .000 |
| Age | .688 | 32.141 | .000 |
| Past history Diabetes | .275 | 12.859 | .000 |
| Past history IHD | .217 | 10.112 | .002 |
| Killip class>1 | .176 | 8.198 | .004 |
| WMSI (wall motion score) | .119 | 5.554 | .019 |
| Female gender | .112 | 5.242 | .022 |
| Diastolic BP | .100 | 4.649 | .031 |
| Past history Hypertension | .071 | 3.332 | NS |
| Heart Rate | .011 | .499 | NS |
| | | | |
| | Adjusted R² 0.38 | | |

Univariate general linear model showing independent predictors of PTA levels

### Day curves for PTA

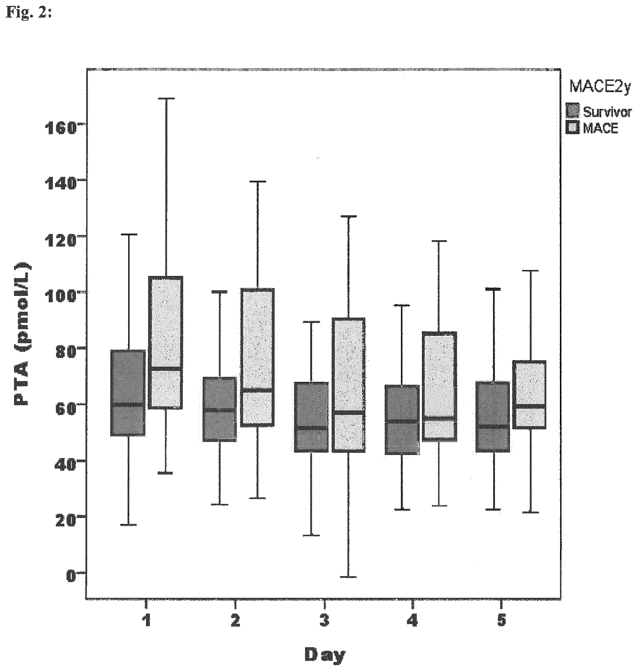

Sequential plasma samples for 5 days were available for 110 patients, of which 29 had a MACE within 2 years. Figure 2 demonstrates the plasma profile along with a general linear model with repeated measures that shows significant changes in PTA over time (p<0.001), and higher levels in those with MACE p<0.03). In post-hoc testing, PTA levels on day 1 was higher than days 3, 4 or 5 (p<0.001, 0.004 and 0.002 respectively, Bonferroni corrected for multiple comparisons). PTA levels on days 1 and 2 were similar. There was no statistically significant interaction of the time profile of PTA with MACE.

### Survival analysis

During follow-up over 2 years, patients with elevated PTA levels (log10 transformed and expressed as a continuous variable) had more MACE, deaths, and rehospitalizations with HF or re-AMI (Table 2). Table 4 reports the univariate and multivariate hazard ratios of various factors that affected the outcome of MACE at 2 years.

**Table 4:**

| | **Univariate HR (95% CI)** | **P** | **Multivariate Model 1 HR (95% CI)** | **P** | **Multivariate Model 2 HR (95% CI)** | **P** | **Multivariate Model 3 HR (95% CI)** | **P** |
|---|---|---|---|---|---|---|---|---|
| Age | 1.05 (1.04-1.06) | 0.000 | 1.02 (1.00-1.03) | 0.023 | 1.02 (1.00-1.03) | 0.027 | 1.02 (1.00-1.04) | 0.024 |
| Gender | 0.63 (0.50-0.79) | 0.000 | | NS | | NS | | NS |
| ST elevation | 0.43 (0.88-1.36) | NS | | | | | | |
| GRACE score | 1.02 (1.02-1.03) | 0.000 | | | | | | |
| Killip class>1 | 2.68 (2.12-3.37) | 0.000 | 1.63 (1.26-2.11) | 0.000 | | NS | 1.75 (1.27-2.42) | 0.001 |
| eGFR | 0.97 (0.96-0.98) | 0.000 | | NS | | NS | | NS |
| Troponin | 1.15 (0.98-1.34) | NS | | | | | | |
| LV systolic dysfunction (echo) | 2.24 (1.74-2.88) | 0.000 | | | | | | |

| **Past history** | | | | | | | | |
|---|---|---|---|---|---|---|---|---|
| Ischemic heart disease | 1.52 (1.22-1.90) | 0.000 | | NS | | NS | | NS |
| Hypertension | 1.64 (1.31-2.05) | 0.000 | | NS | | NS | | NS |
| Diabetes | 1.55 (1.22-1.96) | 0.000 | | NS | | NS | | NS |

| **Treatment on discharge** | | | | | | | | |
|---|---|---|---|---|---|---|---|---|
| Aspirin | 0.57 (0.44-0.75) | 0.000 | | NS | | NS | | NS |
| Beta Blockers | 0.52 (0.41-0.66) | 0.000 | | NS | | NS | 0.64 (0.47-0.88) | 0.006 |
| ACE inhibitor/ARB | 0.55 (0.43-0.71) | 0.000 | 0.74 (0.56-0.99) | 0.045 | 0.65 (0.48-0.89) | 0.007 | | NS |
| S tatins | 0.40 (0.31-0.52) | 0.000 | 0.66 (0.47-0.92) | 0.014 | 0.61 (0.43-0.88) | 0.008 | 0.54 (0.38-0.78) | 0.001 |
| Diuretics | 2.36 (1.89-2.94) | 0.000 | 1.34 (1.03-1.74) | 0.03 | | NS | 1.65 (1.21-2.26) | 0.002 |

| **Biomarkers** | | | | | | | | |
|---|---|---|---|---|---|---|---|---|
| Log NTproBNP | 2.32 (1.90-2.84) | 0.000 | | NS | | NS | 1.74 (1.25-2.42) | 0.001 |
| Log PTA | 19.96 (12.29-32.42) | 0.000 | 3.87 (1.82-8.22) | 0.000 | 6.22 (2.82-13.72) | 0.000 | 4.10 (1.69-9.94) | 0.002 |

Cox regression analysis for MACE at 2 years post-AMI. Multivariate analysis results are reported for model 1 which included variables and biomarkers which were significant on univariate analysis for prediction of MACE (except GRACE and LV systolic dysfunction). Multivariate Models 2 and 3 are for prediction of composite endpoints of death and/or re-MI (model 2) and death and/or HF readmission (model 3). Only significant hazard ratios are reported.

In multivariate analysis for predicting MACE at 2 years, significant independent predictors included age, Killip class above 1, therapy with statins, ACE/ARB and diuretics, and PTA. In other models for prediction of the secondary composite endpoints of death and/or re-AMI (model 2) and death and/or HF readmission (model 3), PTA remained an independent predictor (p<0.0005 and p<0.002 respectively). In models for MACE and each of these secondary composite endpoints which included echocardiographic evidence of LVSD (data not shown), PTA remained a significant independent predictor (P<0.005 for all) together with LVSD (p values ranging from 0.015 to 0.025).

**Table 5 reports the univariate and multivariate hazard ratios for the endpoint of re-AMI at 2 years.**

| | **Univariate HR (95% CI)** | **P** | **Multivariate Model 1 HR (95% CI)** | **P** | **Multivariate Model 2 HR (95% CI)** | **P** | **Multivariate Model 3 HR (95% CI)** | **P** |
|---|---|---|---|---|---|---|---|---|
| Age | 1.02 (1.01-1.03) | 0.004 | | NS | | NS | | NS |
| Gender | 0.85 (0.60-1.21) | NS | | | | | | |
| ST elevation | 1.15 (0.83-1.58) | NS | | | | | | |
| GRACE score | 1.01 (1.00-1.01) | 0.003 | | | | | | |
| Killip class>1 | 1.64 (1.18-2.27) | 0.003 | | NS | | NS | | NS |
| eGFR | 0.99 (0.98-0.99) | 0.016 | | NS | | NS | | NS |
| Troponin | 1.01 (0.81-1.27) | NS | | | | | | |
| LV systolic dysfunction (echo) | 1.60 (1.12-2.29) | 0.010 | | | | | | NS |

| **Past history** | | | | | | | | |
|---|---|---|---|---|---|---|---|---|
| Ischemic heart disease | 1.73 (1.25-2.39) | 0.001 | 1.42 (0.99-2.03) | 0.055 | | NS | | NS |
| Hypertension | 1.56 (1.12-2.18) | 0.008 | | NS | | NS | | NS |
| Diabetes | 1.60 (1.13-2.26) | 0.008 | | NS | | NS | | NS |

| **Treatment on discharge** | | | | | | | | |
|---|---|---|---|---|---|---|---|---|
| Aspirin | 0.99 (0.63-1.55) | NS | | | | | | |
| Beta Blockers | 1.29 (0.82-2.03) | NS | | | | | | |
| ACE inhibitor/ARB | 0.74 (0.50-1.09) | NS | | | | | | |
| S tatins | 1.03 (0.61-1.76) | NS | | | | | | |
| Diuretics | 1.51 (1.07-2.13) | 0.018 | | NS | | NS | | NS |

| **Biomarkers** | | | | | | | | |
|---|---|---|---|---|---|---|---|---|
| Log NTproBNP | 1.34 (1.05-1.71) | 0.019 | | NS | | NS | | NS |
| Log PTA | 4.45 (2.00-9.91) | 0.000 | | | 3.11 (1.06-9.11) | 0.039 | 3.06 (0.92-10.13) | 0.068 |

Table 5: Cox regression analysis for re-MI endpoint at 2 years post-AMI. Multivariate analysis results are reported for model 1 which included variables and NTproBNP which were significant on univariate analysis (except GRACE and LV systolic dysfunction). Multivariate Model 2 reports hazard ratios following addition of pro-SP to model 1. Multivariate Model 3 reports hazard ratios following addition of echocardiographic LVSD to model 2. Only significant hazard ratios are reported.

In model 1 which included significant univariate predictors and NTproBNP, there was a trend to significance (p=0.055) for past history of IHD. Addition of PTA to this revealed that PTA was a significant predictor of re-AMI (model 2, Table 5). When echocardiographic LVSD was added, PTA showed a trend to significance (model 3, Table 5).

### Comparison with GRACE scores

The widely used GRACE risk score (Eagle KA, Lim MJ, Dabbous OH, et al. A validated prediction model for all forms of acute coronary syndrome: estimating the risk of 6-month post discharge death in an international registry. JAMA 2004;291:2727-33.) was originally derived for prediction of death and/or MI at 6 months. GRACE scores and the biomarkers NTproBNP and PTA were predictors of MACE in univariate analysis (Table 6).

**Table 6:**

| | **Univaria te HR (95% CI)** | **P** | **Multivaria te Model 1 HR (95% CI)** | **P** | **Multivaria te Model 2 HR (95% CI)** | **P** | **Multivaria te Model 3 HR (95% CI)** | **P** |
|---|---|---|---|---|---|---|---|---|
| GRACE score | 1.02 (1.02-1.03) | 0.00 0 | 1.02 (1.01-1.02) | 0.00 0 | 1.01 (1.01-1.02) | 0.00 0 | 1.02 (1.02-1.03) | 0.00 0 |

| **Biomarke rs** | | | | | | | | |
|---|---|---|---|---|---|---|---|---|
| Log NTproBN P | 2.50 (1.97-3.18) | 0.00 0 | 1.39 (1.05-1.83) | 0.02 | | NS | 2.21 (1.46-3.35) | 0.00 0 |
| Log PTA | 17.19(9.8 5-29.99) | 0.00 0 | 3.90 (1.72-8.84) | 0.00 1 | 5.93 (2.64-13.33) | 0.00 0 | 3.46 (1.29-9.28) | 0.01 4 |

Cox regression analysis for endpoints at 6 months. Univariate HR refers to MACE at 6 months. Multivariate models included GRACE score and biomarkers for prediction of MACE (model 1), death and/or MI (model 2) and death and/or HF (model 3). Only significant hazard ratios are reported.

In multivariate analysis for MACE, death and/or MI and death and/or HF at 6 months, GRACE score and PTA remained predictors, whereas NTproBNP was only retained for the MACE and death and/or HF model (models 1 and 3 in Table 6).

Using receiver operating characteristic curve (ROC) analysis for death and/or MI at 6 months, the area under the curve (AUC) increased from 0.695 (95% CI 0.649-0.741) for GRACE scoring only to 0.722 (0.677-0.768) with the addition of PTA (P=0.016 compared to GRACE scoring alone). With the addition of NTproBNP to this ROC, there was no further increase of the AUC (0.722 (0.676-0.768)), although this AUC was significantly larger than that of GRACE scoring alone (P=0.027).

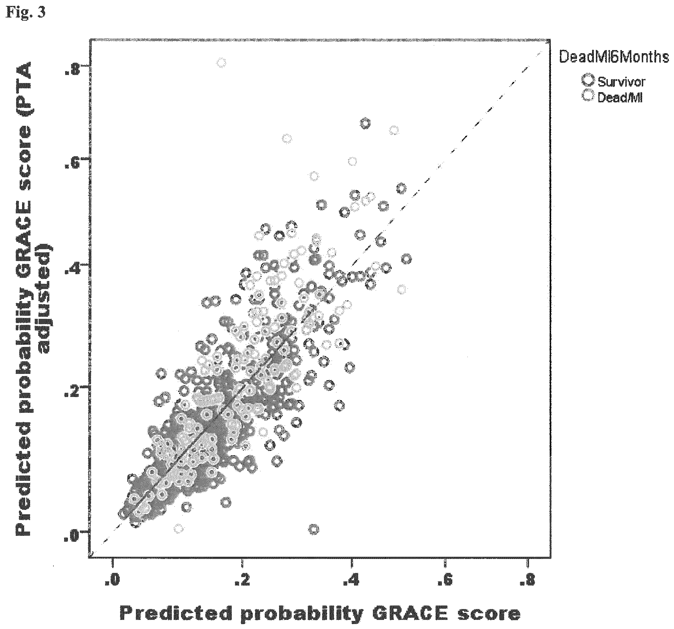

Category-free reclassification analysis was employed as described by Pencina et al (Pencina MJ, D'Agostino RB Sr, Steyerberg EW. Extensions of net reclassification improvement calculations to measure usefulness of new biomarkers. Stat Med. 2011; 30:11-21.) to calculate the NRI (>0) so that no arbitrary cut-off probabilities are chosen for analysis. Figure 3 shows the reclassification plot as proposed by Steyerberg (Steyerberg EW, Vickers AJ, Cook NR, Gerds T, Gonen M, Obuchowski N, Pencina MJ, Kattan MW. Assessing the performance of prediction models. A framework for traditional and novel measures. Epidemiology 2010; 21:128--138.), with probabilities of events using GRACE scoring only plotted against probabilities of events using PTA adjusted GRACE scores. Probabilities adjusted by PTA in those without adverse outcome are down-classified (ie. below the diagonal in figure 3). The NRI in those without the endpoint of death and/or MI at 6 months was 22.3 (95% CI 15.6 - 29.0, P<0.0005) and in those with the endpoint was 9.3 (95%CI -6.7- 25.3, P=NS), with the overall NRI (>0) of 31.6 (95%CI 14.3 - 49.0, P<0.0005), suggesting PTA improved the risk stratification from GRACE scoring predominantly by downclassifying those without endpoints (Table 7).

**Table 7:**

| Continuous | | | | | | |
|---|---|---|---|---|---|---|
| | **Up** | **Down** | **NRI** | **p** | **LowerCI** | **UpperCI** |
| **Without endpoint** | 329 | 518 | 22.3 | .000 | 15.6 | 29.0 |
| **With endpoint** | 82 | 68 | 9.3 | .253 | -6.7 | 25.3 |
| **Total** | | | 31.6 | .000 | 14.3 | 49.0 |

| Tertiles | | | | | | |
|---|---|---|---|---|---|---|
| | **Up** | **Down** | **NRI** | **p** | **LowerCI** | **UpperCI** |
| **Without endpoint** | 86 | 135 | 5.8 | .001 | 2.3 | 9.2 |
| **With endpoint** | 21 | 15 | 4.0 | .317 | -3.8 | 11.8 |
| **Total** | | | 9.8 | .025 | 1.2 | 18.3 |

| Risk threshold 0.05 and 0.1 | | | | | | |
|---|---|---|---|---|---|---|
| | **Up** | **Down** | **NRI** | **p** | **LowerCI** | **UpperCI** |
| **Without endpoint** | 58 | 157 | 11.7 | .000 | 8.3 | 15.1 |
| **With endpoint** | 6 | 10 | -2.7 | .317 | -7.9 | 2.6 |
| **Total** | | | 9.0 | .005 | 2.8 | 15.3 |

Reclassification analysis using continuous reclassification, tertiles or 2 specified risk thresholds of 0.05 and 0.1, showing the net reclassification improvement (NRI) and the significance of the NRI, of adding PTA to the classification using GRACE scoring only, for the endpoint of death and/or MI at 6 months.

When tertiles are chosen as the two cut-offs for probabilities of death and/or MI at 6 months, reclassification analysis showed that PTA mainly down classified those without the endpoint, with a significant net NRI (9.8, P<0.025).

When two cut-offs for probabilities of death and/or MI at 6 months (5% and 10%) are chosen, the NRI in those without the endpoint of death and/or MI at 6 months was 11.7 (P<0.0005), and a non-significant NRI in those with the endpoint, leading to an overall NRI of 9.0 (P<0.005).

### Decision tree analysis

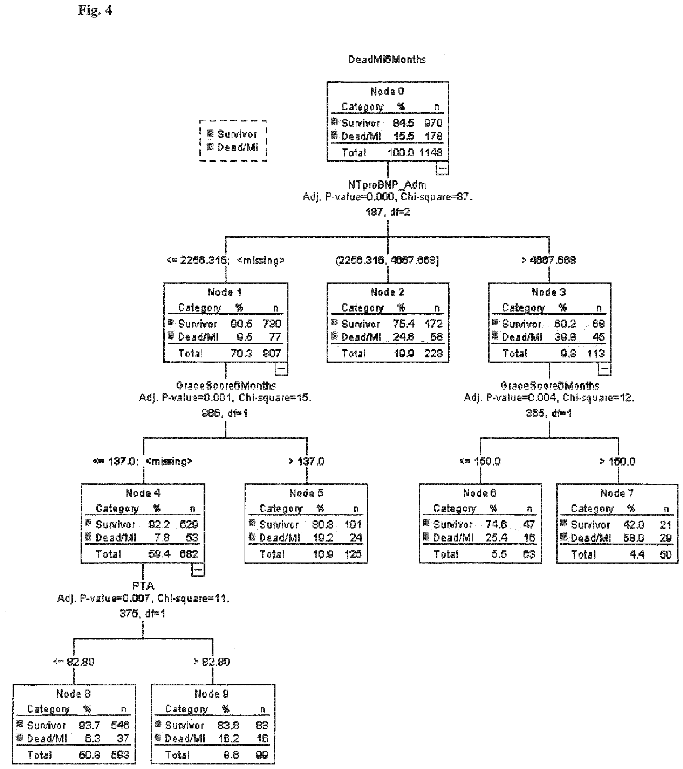

In order to determine optimal cut-points for biomarkers, we constructed decision trees (using PTA and NTproBNP levels and GRACE scores) to classify patients into survivors or those with endpoints. For the endpoint of death and/or MI at 6 months (figure 4), PTA under 82.8 pmol/L selected a subgroup (n=583, 50.8% of the total) in those who had NTproBNP under 2256 pmol/L and GRACE score under 137 who had a low risk of death and/or MI (6.3% compared to 15.5% in the original population).

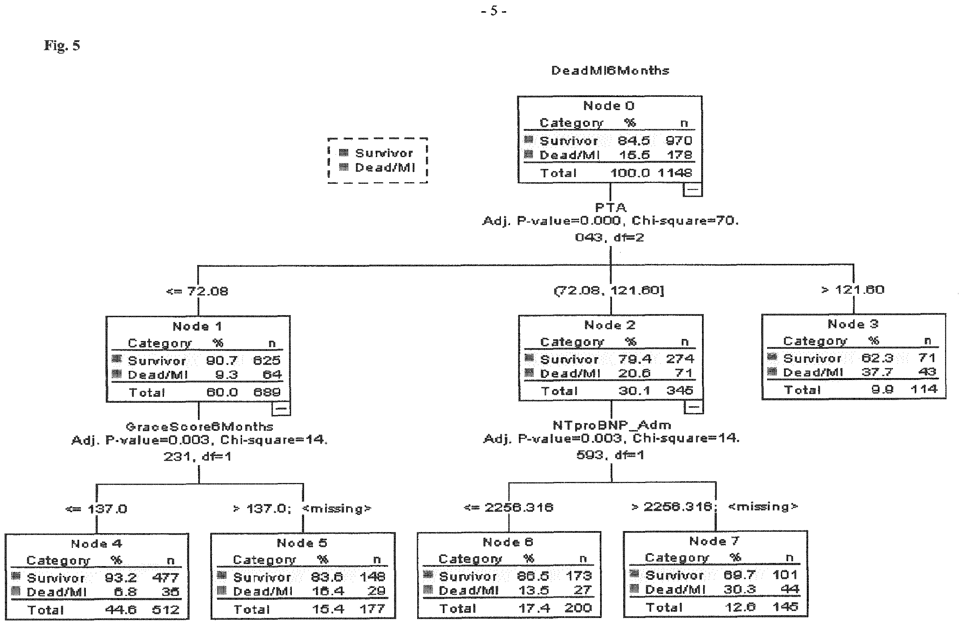

In decision trees using PTA as an initial classifier (figure 5) a PTA level under 72.08 pmol/L and GRACE score under 137 defines a low risk group of patients (n=512, 44.6% of the total) in whom the event rate was 6.8%. Of these, only 3 patients (0.26%) had died within 6 months, and 1 (0.09%) had died within 30 days. PTA levels above 121.6 pmol/L defined a high risk group of patients with a death/MI rate of 37.7% and a death rate of 30.7% (figure 5).

### Kaplan Meier analysis

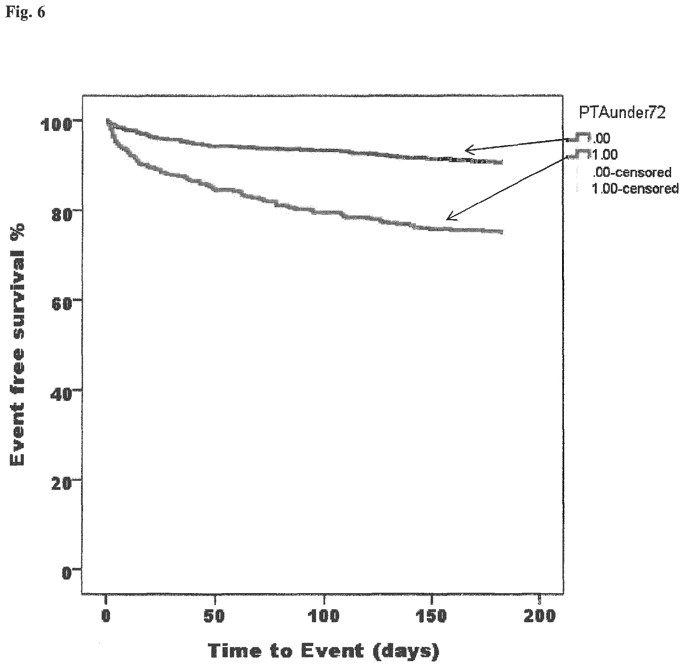

Using a cut-off value of 72.1 pmol/L, Kaplan Meier survival analysis for the combined endpoint of death and/or MI demonstrated a significant difference between those with levels below (n=689, 60% of the total) or above this cut-off (P value for log rank test <0.0005) (figure 6).

## Claims

1. A method for predicting the risk of getting a major adverse cardiac event or death in a subject that has suffered an acute myocardial infarction comprising:
• determining the level of Protachykinin according to SEQ ID NO. 1, SEQ ID NO. 8, SEQ ID NO. 10, SEQ ID NO. 11 or fragments thereof in a bodily fluid obtained from said subject; and
• correlating said level of Protachykinin SEQ ID NO. 1, SEQ ID NO. 8, SEQ ID NO. 10, SEQ ID NO. 11 or fragments thereof with the risk for getting a major adverse cardiac event or death, wherein an elevated level is predictive for an enhanced risk of getting a major adverse cardiac event or death,
wherein fragments of Protachykinin are selected from the group comprising SEQ ID NO. 2, SEQ ID NO. 12, and SEQ ID NO. 13, and
wherein said bodily fluid is selected from the group comprising blood, serum, and plasma.

2. A method according to claim 1, wherein the level of Protachykinin or fragments thereof in a bodily fluid obtained from said subject is determined, wherein at least one binder is used for said determination that binds to PTA 1-37, SEQ ID NO. 2, EEIGANDDLNYWSDWYDSDQIKEELPEPFEHLLQRIA, and wherein said binder has an affinity to PTA 1-37 of at least 10⁷ M⁻¹.

3. A method according to claim 1 or 2, wherein said major adverse cardiac event is an acute major adverse cardiac event selected from the group comprising myocardial infarction, stroke, and acute heart failure.

4. A method according to any of claims 1 to 3, wherein a sample of bodily fluid from said subject has been taken within a certain time frame after the AMI has occurred, this time frame being 2 months, more preferably 1 month, more preferably 1 week, most preferably within 24 hours.

5. A method for predicting the risk of getting a major adverse cardiac event or death in a subject that has suffered an acute myocardial infarction according to any of claims 1 to 4, wherein an elevated level is predictive for an enhanced risk of getting a major adverse cardiac event or death within the next 6 months or within the next 2 years.

6. A method according to any of claims 1 to 5, wherein in addition the level of one or more of the following marker is determined and used: Troponin I, Troponin T, CRP, LpLA2, Cystatin C and natriuretic peptides of the A- and the B-type as well as their precursors and fragments thereof including ANP, proANP, NT-proANP, MR-proANP, BNP, proBNP, NT-proBNP triglycerides, HDL cholesterol or subfractions thereof, LDL cholesterol or subfractions thereof, GDF15, ST2, copeptin, and/or any score, such as for instance the PURSUIT, TIMI, GRACE and FRISC risk score.

7. A method according to any of claims 1 to 6, wherein additionally at least one clinical parameter is determined selected from the group comprising: age, systolic blood pressure, diastolic blood pressure, antihypertensive treatment, body mass index, presence of diabetes mellitus, current smoking.

8. A method according to any of the preceding claims, wherein the level of Protachykinin or fragments thereof is measured with an immunoassay.

9. A method according to any of claims 1 - 8, wherein said method is performed more than once in order to monitor the risk of getting a major adverse cardiac event or death in a subject.

10. A method according to claim 9, wherein said monitoring is performed in order to evaluate the response of said subject to preventive and/or therapeutic measures taken.

11. A method according to any of claims 1 to 10 in order to stratify said subjects into risk groups.

12. Use of a device selected from the group comprising sandwich assays, fully automated or manual assays, ELISAs, and point-of-care devices for performing a method according to any of claims 2 to 11.

13. Use of a device according to claim 12, comprising two binders that bind to two different regions within the region of PTA, wherein said two regions correspond to at least 4 or 5 amino acids of SEQ ID NO. 12 and 13.

14. Use of a device according to claim 12, further comprising:
• a binder against proBNP or fragments or precursors thereof having at least 5 amino acids, and
• a binder against Protachykinin or fragments thereof of at least 5 amino acids or Protachykinin-comprising peptides and/or a binder against CRP.

15. Use of a device according to claim 14, wherein said binder is selected from the group comprising an antibody, an antibody fragment and a non-Ig scaffold.

## Patentansprüche

1. Verfahren zur Vorhersage des Risikos für ein schwerwiegendes unerwünschtes kardiales Ereignis oder Tod bei einem Subjekt, das einen akuten Myokardinfarkt erlitten hat, umfassend:
• die Bestimmung des Wertes von Protachykinin gemäß SEQ ID NO. 1, SEQ ID NO. 8, SEQ ID NO. 10, SEQ ID NO. 11 oder Fragmenten davon in einer von diesem Subjekt entnommenen Körperflüssigkeit; und
• die Korrelation dieses Wertes von Protachykinin SEQ ID NO. 1, SEQ ID NO. 8, SEQ ID NO. 10, SEQ ID NO. 11 oder Fragmenten davon mit dem Risiko für ein schwerwiegendes unerwünschtes kardiales Ereignis oder Tod, wobei ein erhöhter Wert einem erhöhten Risiko für ein schwerwiegendes unerwünschtes kardiales Ereignis oder Tod entspricht,
wobei Fragmente von Protachykinin ausgewählt sind aus der Gruppe umfassend SEQ ID NO. 2, SEQ ID NO. 12 und SEQ ID NO. 13, und
wobei die Körperflüssigkeit ausgewählt ist aus der Gruppe umfassend Blut, Serum und Plasma.

2. Verfahren nach Anspruch 1, wobei der Wert von Protachykinin oder Fragmenten davon in einer dem Subjekt entnommenen Körperflüssigkeit bestimmt wird, wobei für diese Bestimmung mindestens ein Binder verwendet wird, der an PTA 1-37, SEQ ID NO. 2, EEIGANDDLNYWSDWYDSDQIKEELPEPFEHLLQRIA bindet, und wobei der Binder eine Affinität zu PTA 1-37 von mindestens 10⁷ M⁻¹ aufweist.

3. Verfahren nach Anspruch 1 oder 2, wobei das schwerwiegende unerwünschte kardiale Ereignis ein akutes schwerwiegendes unerwünschtes kardiales Ereignis, ausgewählt aus der Gruppe umfassend Myokardinfarkt, Schlaganfall und akuter Herzinsuffizienz, ist.

4. Verfahren nach einem der Ansprüche 1 bis 3, wobei eine Probe der Körperflüssigkeit des Subjekts innerhalb einer bestimmten Zeitspanne nach Eintreten des AMI entnommen wurde, wobei diese Zeitspanne 2 Monate, bevorzugt 1 Monat, weiter bevorzugt 1 Woche und am meisten bevorzugt 24 Stunden beträgt.

5. Verfahren zur Vorhersage des Risikos für ein schwerwiegendes unerwünschtes kardiales Ereignis oder Tod in einem Subjekt, das einen akuten Myokardinfarkt erlitten hat, nach einem der Ansprüche 1 bis 4, wobei ein erhöhter Wert einem erhöhten Risiko für ein schwerwiegendes unerwünschtes kardiales Ereignis oder Tod innerhalb der nächsten 6 Monate oder innerhalb der nächsten 2 Jahre entspricht.

6. Verfahren nach einem der Ansprüche 1 bis 5, wobei zusätzlich der Wert eines oder mehrerer der folgenden Marker bestimmt und verwendet wird: Troponin I, Troponin T, CRP, LpLA2, Cystatin C und natriuretische Peptide vom Typ A und Typ B sowie deren Vorstufen und Fragmente davon, einschließlich ANP, proANP, NT-proANP, MR-proANP, BNP, proBNP, NT-proBNP Triglyceride, HDL-Cholesterin oder Subfraktionen davon, LDL-Cholesterin oder Subfraktionen davon, GDF15, ST2, Copeptin und/oder ein beliebiger Score wie beispielsweise der PURSUIT-, TIMI-, GRACE- und FRISC-Risiko-Score.

7. Verfahren nach einem der Ansprüche 1 bis 6, wobei zusätzlich mindestens ein klinischer Parameter, ausgewählt aus der Gruppe umfassend Alter, systolischem Blutdruck, diastolischem Blutdruck, Behandlung mit Antihypertensiva, Body-Mass-Index, Vorhandensein von Diabetes mellitus, derzeitigen Rauchgewohnheiten, bestimmt wird.

8. Verfahren nach einem der vorangehenden Ansprüche, wobei der Wert von Protachykinin oder von Fragmenten davon mithilfe eines Immunoassays bestimmt wird.

9. Verfahren nach einem der Ansprüche 1 bis 8, wobei dieses Verfahren mehr als einmal durchgeführt wird, um das Risiko für ein schwerwiegendes unerwünschtes kardiales Ereignis oder Tod bei einem Subjekt zu überwachen.

10. Verfahren nach Anspruch 9, wobei die Überwachung durchgeführt wird, um die Reaktion des Subjekts auf getroffene vorbeugende und/oder therapeutische Maßnahmen zu bewerten.

11. Verfahren nach einem der Ansprüche 1 bis 10, um die Subjekte in Risikogruppen zu stratifizieren.

12. Verwendung einer Vorrichtung ausgewählt aus der Gruppe umfassend Sandwich-Assays, vollautomatischen oder manuellen Assays, ELISAs und Point-of-Care-Geräten zur Durchführung eines Verfahrens nach einem der Ansprüche 2 bis 11.

13. Verwendung einer Vorrichtung nach Anspruch 12, welches zwei Binder umfasst, die an zwei verschiedene Regionen innerhalb der PTA-Region binden, wobei diese zwei Regionen mindestens 4 oder 5 Aminosäuren der SEQ ID NO. 12 und 13 entsprechen.

14. Verwendung einer Vorrichtung nach Anspruch 12, weiterhin umfassend:
• ein Binder gegen proBNP oder Fragmente oder Vorstufen davon mit mindestens 5 Aminosäuren und
• ein Binder gegen Protachykinin oder Fragmente davon mit mindestens 5 Aminosäuren oder Protachykinin-umfassende Peptide und/oder Binder gegen CRP.

15. Verwendung einer Vorrichtung nach Anspruch 14, wobei das Bindemittel ausgewählt ist aus der Gruppe umfassend ein Antikörper, ein Antikörperfragment und ein nicht-Immunglobulin-Gerüst.

## Revendications

1. Méthode de prédiction du risque de survenue d'un incident cardiaque indésirable majeur ou de mort chez un sujet atteint d'un infarctus aigu du myocarde, ladite méthode comprenant:
• déterminer le taux de protachykinine selon SEQ ID NO. 1, SEQ ID NO. 8, SEQ ID NO. 10, SEQ ID NO. 11 ou de ses fragments dans un fluide corporel prélevé chez ledit sujet; et
• corréler ledit taux de protachykinine de SEQ ID NO. 1, SEQ ID NO. 8, SEQ ID NO. 10, SEQ ID NO. 11 ou de ses fragments avec le risque de survenue d'un incident cardiaque indésirable majeur ou de mort, un taux élevé étant indicateur d'un risque accru de survenue d'un incident cardiaque indésirable majeur ou de mort,
les fragments de protachykinine étant choisis dans le groupe comprenant SEQ ID NO. 2, SEQ ID NO. 12, et SEQ ID NO. 13, et
ledit fluide corporel étant choisi dans le groupe comprenant le sang, le sérum et le plasma.

2. Méthode selon la revendication 1, le taux de protachykinine ou de ses fragments dans un fluide corporel prélevé chez ledit sujet étant déterminé, au moins un liant étant utilisé pour ladite détermination qui lie PTA 1-37, SEQ ID NO. 2, EEIGANDDLNYWSDWYDSDQIKEELPEPFEHLLQRIA, et ledit liant ayant une affinité pour PTA 1-37 d'au moins 10⁷ M⁻¹.

3. Méthode selon la revendication 1 ou 2, ledit incident cardiaque indésirable majeur étant un incident cardiaque indésirable majeur aigu choisi dans le groupe comprenant l'infarctus du myocarde, l'AVC et l'insuffisance cardiaque aiguë.

4. Méthode selon une quelconque des revendications 1 à 3, un échantillon de fluide corporel dudit sujet ayant été prélevé dans une certain plage de temps après l'IAM, cette plage de temps étant de 2 mois, de préférence 1 mois, de manière plus préférée 1 semaine, de manière encore plus préférée dans les 24 heures.

5. Méthode de prédiction du risque de survenue d'un incident cardiaque indésirable majeur ou de mort chez un sujet atteint d'un infarctus aigu du myocarde selon une quelconque des revendications 1 à 4, un taux élevé étant prédictif d'un risque accru de survenue d'un incident cardiaque indésirable majeur ou de mort dans les 6 prochains mois ou 2 prochaines années.

6. Méthode selon une quelconque des revendications 1 à 5, en outre le taux d'un ou plusieurs des marqueurs suivants étant déterminé et utilisé : Troponine I, Troponine T, CRP, LpLA2, Cystatine C et peptides natriurétiques de type A et B ainsi que leurs précurseurs et fragments, y compris ANP, proANP, NT-proANP, MR-proANP, BNP, proBNP, triglycérides NT-proBNP, cholestérol HDL ou leurs sous-fractions, cholestérol LDL ou leurs sous-fractions, GDF15, ST2, copéptine, et/ou tout score, comme par exemple la cote de risque PURSUIT, TIMI, GRACE et FRISC.

7. Méthode selon une quelconque des revendications 1 à 6, en outre au moins un paramètre clinique déterminé étant choisi parmi le groupe comprenant : âge, tension artérielle systolique, tension artérielle diastolique, traitement antihypertenseur, indice de masse corporelle, présence du diabète sucré, habitudes de tabagisme actuelles.

8. Méthode selon une quelconque des revendications précédentes, le taux de protachykinine ou de ses fragments étant mesuré à l'aide d'un immunoessai.

9. Méthode selon une quelconque des revendications 1 à 8, ladite méthode étant effectuée plus d'une fois pour surveiller le risque de survenue d'un incident cardiaque indésirable majeur ou de mort chez un sujet.

10. Méthode selon la revendication 9, ladite surveillance étant réalisée pour évaluer la réponse dudit sujet aux mesures préventives et/ou thérapeutiques prises.

11. Méthode selon une quelconque des revendications 1 à 10 pour stratifier lesdits sujets en groupes de risque.

12. Utilisation d'un dispositif choisi dans le groupe comprenant les essais en sandwich, entièrement automatisés ou manuels, les tests ELISA et les dispositifs sur le lieu de soins pour exécuter une méthode selon une quelconque des revendications 2 à 11.

13. Utilisation d'un dispositif selon la revendication 12, comprenant deux liants qui se lient à deux régions différentes dans la région de PTA, lesdites deux régions correspondant à au moins 4 ou 5 acides aminés de SEQ ID N° 12 et 13.

14. Utilisation d'un dispositif selon la revendication 12, comprenant en outre:
• un liant contre le proBNP ou ses fragments ou précurseurs ayant au moins 5 acides aminés, et
• un liant contre la protachykinine ou ses fragments d'au moins 5 acides aminés ou des peptides comprenant la protachykinine et/ou un liant contre la CRP.

15. Utilisation d'un dispositif selon la revendication 14, ledit liant étant choisi parmi le groupe comprenant un anticorps, un fragment d'anticorps et un échafaudage non Ig.
